# FASCICULE DE BREVET EUROPEEN

(11) **EP 4 446 010 B1**
(45) Date de publication et mention de la délivrance du brevet: **22.10.2025**
(21) Numéro de dépôt: 24162092.1
(22) Date de dépôt: 07.03.2024
(51) Int. Cl.: B01L 3/00, C12M 1/14, C12M 1/16, C12Q 1/04, C12Q 1/18, G01N 33/569, C12Q 1/70

(54) **PROCÉDÉ DE DÉTECTION D'UNE SENSIBILITÉ DE PHAGES OU D'ANTIBIOTIQUES VIS-À-VIS D'UNE SOUCHE BACTÉRIENNE**
VERFAHREN ZUM NACHWEIS DER EMPFINDLICHKEIT VON PHAGEN ODER ANTIBIOTIKA GEGENÜBER BAKTERIENSTAMM
METHOD FOR DETECTING PHAGE OR ANTIBIOTIC SUSCEPTIBILITY TO A BACTERIAL STRAIN

(30) Priorité: 14.03.2023 FR 2302364
(43) Date de publication de la demande: 16.10.2024
(73) Titulaire: Commissariat à l'Energie Atomique et aux Energies Alternatives, 75015 Paris (FR); Université Grenoble Alpes, 38400 Saint-Martin-d'Hères (FR); Centre National de la Recherche Scientifique, 75016 Paris Cedex 16 (FR); Institut Polytechnique de Grenoble, 38000 Grenoble (FR)
(72) Inventeur: MARCOUX, Pierre, 38054 Grenoble Cedex 09 (FR); ROUPIOZ, Yoann, 38570 Theys (FR); ZELSMANN, Marc, 38330 Biviers (FR); PICARD, Emmanuel, 38054 Grenoble cedex 09 (FR); O'CONNELL, Larry, Londres, SW7 2AZ (GB)
(74) Mandataire: INNOV-GROUP

(56) Documents cités:
- EP-A1- 3 822 360
- WO-A1-2011/009213
- WO-A1-2018/102346

## Description

### Domaine technique de l'invention

La présente invention se rapporte à un procédé de détection sensibilité de phages ou d'antibiotiques vis-à-vis d'une souche bactérienne.

### Etat de la technique

La phagothérapie est une approche médicale utilisée pour traiter les maladies infectieuses d'origine bactérienne, s'appuyant sur les capacités naturelles de certains virus, appelés bactériophages (et couramment appelés phages), à détruire les bactéries qu'ils reconnaissent spécifiquement.

La mise en œuvre de cette approche nécessite au préalable de sélectionner les phages qui présentent une sensibilité particulière pour la bactérie à détruire. Une méthode classique consiste à utiliser une plaque à puits, dans laquelle chaque puits est rempli par une suspension de bactéries. Des phages distincts en composition et/ou en concentration sont ensuite ajoutés dans chaque puits. On surveille ensuite dans chaque puits si une réaction de lyse (destruction) se produit ou non. Lorsqu'une lyse se produit dans un puits, on peut en déduire que le phage ajouté à la concentration définie présente une affinité et une activité lytique pour la bactérie. Il sera donc potentiellement utilisable pour la mise en place d'une phagothérapie.

La demande de brevet EP3822360A1 décrit une solution utilisant un principe de détection d'activité lytique de phages par imagerie sans lentille. Un mode de réalisation particulier consiste à placer une carte fluidique dotée de plusieurs chambres entre une source de lumière et un capteur d'image. Dans un exemple de réalisation, on place dans chaque chambre des bactéries d'une même souche bactérienne et on vient y ajouter des phages de souches virales distinctes, ou d'une même souche virale mais à des concentrations distinctes. On observe ensuite la réaction qui se produit dans chaque chambre. Grâce au capteur d'image, on détermine si la bactérie est :
- Peu ou pas sensible à la souche virale du phage lorsque l'intensité lumineuse détectée diminue entre deux instants de mesure successifs, ou
- Sensible à la souche virale du phage lorsque l'intensité lumineuse détectée ne diminue pas ou augmente entre deux instants de mesure successifs.

Cette solution antérieure peut présenter des risques de pollution des échantillons, chaque chambre pouvant être exposée à l'environnement extérieur.

La demande de brevet WO2011/009213 concerne une méthode de test d'affinités entre des biofilms et des agents anti-microbiens.

Le but de l'invention est de proposer une solution permettant de détecter une interaction entre une souche bactérienne et des bactériophages ou des antibiotiques distincts en composition/concentration qui soit :
- Automatisée ou facile à automatiser ;
- Simple et fiable à mettre en œuvre, permettant de paralléliser des zones de détection distinctes, limitant les risques de contaminations croisées, tout en ne compromettant pas la croissance bactérienne ;
- Rapide à mettre en œuvre et permettant de tester une grande quantité de combinaisons, en limitant le nombre de manipulations à effectuer ;

Cette invention vise in fine, à identifier, de manière parallélisée, les types de phages doués d'une activité lytique vis à vis d'une bactérie ciblée. Les résultats ainsi produits permettent alors de définir les combinaisons de types de phages ("cocktails de phages"), susceptibles d'avoir une activité thérapeutique in vivo.

L'invention permet ainsi de déterminer l'impact de diverses concentrations d'un panel d'antibactériens (antibiotiques ou bactériophages), sur la biomasse d'une souche bactérienne.

### Exposé de l'invention

Ce but est atteint par un procédé de détection d'une activité lytique d'un ou plusieurs types de phages ou d'activité d'un ou plusieurs antibiotiques vis-à-vis d'une souche bactérienne, les phages ou antibiotiques étant éventuellement distincts entre eux en composition et/ou concentration, le procédé consistant à :
- Utiliser une première plaque à plusieurs puits, chaque puits étant réalisé dans un matériau transparent aux signaux lumineux émis par une source de lumière et perméable aux gaz apolaires, chaque puits recevant un échantillon contenant des phages ou des antibiotiques,
- Réaliser au moins un dépôt sur une zone transparente d'un support, le dépôt contenant des bactéries de la souche bactérienne,
- Appliquer la première plaque contre le support de sorte que chaque échantillon présent dans chaque puits vienne au contact dudit dépôt,
- Disposer l'ensemble formé de la première plaque et le support entre une source de lumière et des moyens de détection de lumière,
- Emettre à l'aide de la source de lumière des signaux lumineux à travers chaque puits,
- Pour chaque puits, déterminer à l'aide des moyens de détection l'activité ou l'absence d'activité du phage ou de l'antibiotique vis-à-vis de ladite souche bactérienne en tenant compte de l'intensité des signaux lumineux transmis et/ou de sa variation au cours du temps.

Selon une particularité, les phages ou antibiotiques sont présents dans chaque puits sous la forme d'un hydrogel.

Selon une autre particularité, les bactéries sont dispersées dans un gel déposé sur ladite deuxième plaque de manière à former ledit dépôt.

Selon une autre particularité, le support est réalisé sous la forme d'une deuxième plaque compartimentée à l'aide de cloisons étanches de manière à former plusieurs compartiments distincts, ledit dépôt étant subdivisé en plusieurs dépôts distincts ajoutés dans chaque compartiment de la deuxième plaque.

Selon une autre particularité, la première plaque est maintenue contre ladite deuxième plaque de sorte que les phages ou antibiotiques contenus dans chaque puits viennent au contact avec un dépôt placé dans un compartiment distinct de la deuxième plaque. Selon une autre particularité, la première plaque est retournée et déplacée pour venir s'appliquer par une face ouverte sur lesdits puits contre une face fonctionnelle du support portant le dépôt.

Selon une autre particularité, le support est retourné de manière à venir s'appliquer par une face fonctionnelle portant ledit dépôt contre une face de la première plaque ouverte sur lesdits puits.

Selon une autre particularité, le procédé comporte une étape de déplacement relatif de l'ensemble formé par la première plaque et le support par rapport aux moyens de détection en vue de transmettre les signaux lumineux à travers chaque puits de la première plaque.

Selon une autre particularité, les moyens de détection comportent un capteur d'images. Selon une autre particularité, le capteur d'images est configuré pour acquérir une première image à un premier instant et une deuxième image à un deuxième instant postérieur et en ce qu'une unité de traitement est configurée pour déterminer que la souche bactérienne est considérée comme :
- Peu ou pas sensible aux phages ou antibiotiques lorsque l'intensité des signaux lumineux détectés diminue entre lesdits deux instants, ou
- Sensible aux phages ou antibiotiques lorsque l'intensité des signaux lumineux détectés ne diminue pas ou augmente entre lesdits deux instants.

Selon une autre particularité, le capteur d'images coopère avec la source de lumière selon un principe d'imagerie sans lentille.

Selon une autre particularité, la source de lumière est configurée pour émettre dans une bande spectrale comprise entre 500nm et 600nm.

### Brève description des figures

D'autres caractéristiques et avantages vont apparaître dans la description détaillée qui suit, en liaison avec les figures annexées listées ci-dessous :
- La figure 1 montre de manière schématique le système de détection de l'invention.
- La figure 2 illustre la mise en œuvre du procédé de détection de l'invention.

### Description détaillée d'au moins un mode de réalisation

L'invention vise un procédé qui est adapté pour :
- Détecter la sensibilité, vis-à-vis de bactéries d'une même souche bactérienne, de phages issus de souches virales distinctes ;
- Détecter la sensibilité, vis-à-vis de bactéries d'une même souche bactérienne, de phages issus d'une même souche virale, à des concentrations différentes ;
- Détecter la sensibilité, vis-à-vis de bactéries d'une même souche bactérienne, de phages issus de souches virales distinctes et à des concentrations différentes ;

Le même principe peut s'appliquer pour des antibiotiques vis-à-vis de bactéries d'une même souche bactérienne. Dans la suite de la description, on évoquera les phages de manière générale, pour évoquer les principes de détection du procédé, définis ci-dessus, mais il faut comprendre que l'invention peut s'appliquer de manière identique à des antibiotiques.

Le procédé de l'invention permet, sur un même dispositif, de mettre en contact des bactéries B avec des suspensions de phages P, distinctes en composition et/ou en concentration, de manière simple, en formant des zones de détection distinctes.

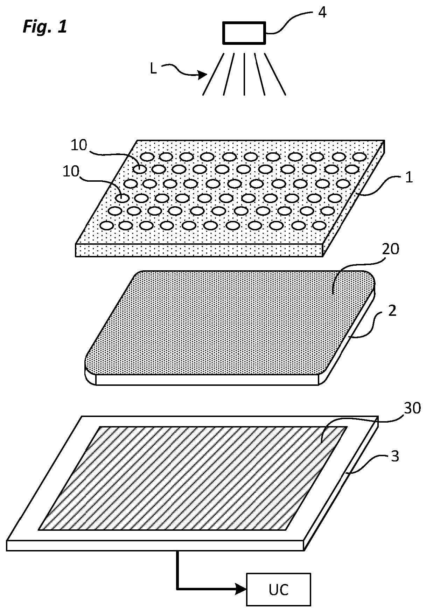

Le procédé est mis en œuvre en utilisant le système de détection visible sur la figure 1.

Ce système comporte une première plaque 1 à puits. Cette première plaque 1 comporte ainsi plusieurs puits 10 juxtaposés. Par "puits", on entend que la plaque 1 comporte plusieurs cavités distinctes, physiquement séparées ou suffisamment espacées les unes des autres.

Au niveau de chacun de ses puits, cette plaque 1 présente la particularité d'être réalisée dans un matériau :
- Transparent, préférentiellement dans le visible et/ou proche infrarouge, pour laisser passer des signaux lumineux ;
- Perméable aux gaz apolaires, notamment au dioxygène et au dioxyde de carbone pour garantir la croissance bactérienne ; On verra que cette caractéristique est importante car elle permet de garantir la croissance bactérienne même lors de la mise en œuvre du procédé de l'invention ;

De manière non limitative, la première plaque 1 est par exemple réalisée dans sa totalité dans un matériau tel que le PDMS (PolyDiMéthyl Siloxane) qui présente les caractéristiques définies ci-dessus. Ce matériau présente également l'avantage d'être biocompatible.

Selon le procédé de l'invention, chaque puits 10 est rempli avec une suspension de phages. D'un puits à un autre, les suspensions de phages P peuvent être distinctes en composition et/ou en concentration.

Il faut noter que la plaque 1 peut se présenter sous la forme d'un dispositif prérempli avec les phages P et ainsi prêt à l'emploi. La plaque 1 est par exemple maintenue scellée en attente de son utilisation.

Les phages P peuvent être dispersées dans un hydrogel ajouté en quantité adaptée dans chaque puits 10 de la plaque 1. Cet hydrogel peut être une matrice réalisée en agar-agar. Comme cette matrice est composée à environ 99% d'eau, les phages P se retrouvent stockés dans un environnement aqueux salin, ce qui aide à maintenir le titre infectieux des phages sur une longue durée.

La plaque 1 peut comporter plusieurs dizaines de puits 10 juxtaposés. Ce nombre pourra notamment dépendre de la taille du capteur 3 utilisé pour la détection.

Comme indiqué ci-dessus, un matériau tel que le PDMS est imperméable à l'eau mais perméables aux gaz apolaires, ce qui permet des échanges gazeux au niveau des puits 10 et donc d'assurer la croissance bactérienne, même lorsque les phages P et les bactéries B sont mis en contact.

Dans le cadre de l'invention, chaque puits 10 matérialise ainsi une zone de détection délimitée et distincte, au travers de laquelle la source de lumière 4 peut transmettre des signaux lumineux.

Le système comporte un support pour un ou plusieurs dépôts contenant des bactéries B de la souche bactérienne. De manière non limitative et dans la suite de la description, ce support peut être formé d'une deuxième plaque 2 pouvant accueillir, sur toute sa surface ou son volume fonctionnel, au moins un dépôt 20 de matière. Le support pourrait être également formé directement par la surface du capteur (voir ci-après).

Le dépôt 20 présente avantageusement une épaisseur et une viscosité suffisantes pour former un tapis. Ce dépôt 20 peut se présenter sous la forme d'un gel contenant les bactéries. Il peut s'agir d'une substance gélatineuse telle que l'agar-agar, qui est couramment utilisée pour cultiver des bactéries. De manière connue, cette substance est fabriquée à partir d'une algue rouge (compatible avec le niveau de transparence requis pour la détection) qui fournit une surface de croissance adaptée pour de nombreuses variétés de bactéries. Bien entendu, il serait possible d'utiliser une autre substance.

Il faut noter qu'il est possible de réaliser un unique dépôt 20 venant recouvrir toute la surface fonctionnelle de la deuxième plaque 2. Il est également possible de réaliser plusieurs dépôts juxtaposés, chaque dépôt étant destiné à être associé à un puits distinct de la première plaque. Pour accueillir chacun de ces dépôts, la deuxième plaque 2 peut disposer de compartiments, les compartiments étant cloisonnés de manière étanche les uns par rapport aux autres. Cette version compartimentée permet avantageusement d'éviter toute diffusion lors de la mise en œuvre du procédé de l'invention. Il faut en effet noter qu'il est nécessaire d'éviter que la réaction localisée au niveau d'un puits vienne se répandre sur un puits adjacent, c'est à dire que des phages contenus dans un puits ne viennent se répandre au-delà de leur puits, en contaminant un puits adjacent.

Chaque dépôt 20 contient une concentration non nulle de bactéries B.

La deuxième plaque 2, ainsi que le dépôt 20, sont choisis transparents, préférentiellement dans le visible et/ou proche infrarouge, aux signaux lumineux L de la source de lumière (voir ci-après) de manière à pouvoir être traversés au moins partiellement par ces signaux lumineux L lors de la mise en œuvre du procédé. De manière non limitative, la deuxième plaque peut également être réalisée en PDMS. Mais d'autres matériaux tels que PMMA ("Polymétacrylate de Méthyle") ou COC ("Cyclic Olefin Copolymer") pourraient être envisagés.

Le système de détection comporte une source de lumière 4. La source de lumière 4 est par exemple composée d'une ou plusieurs diodes électroluminescentes ou diodes laser. Un filtre passe-bande (non représenté - éventuellement intégré à la source) peut être employé en sortie de la source de lumière 4 ou en entrée du capteur d'images 3, pour ajuster la bande spectrale des signaux lumineux émis. De préférence, les signaux lumineux L émis par la source de lumière 4 s'étendent selon une bande spectrale d'illumination du domaine visible et proche infrarouge. Elle est de préférence comprise entre 400nm et 1000nm, de préférence comprise entre 500nm et 600nm.

Le système de détection comporte ainsi un capteur d'images 3. Ce capteur d'images 3 peut être un capteur de type CCD ou CMOS. De manière non limitative, il présente par exemple une résolution de 5344x3516 pixels. Il faut noter que le système ne comporte avantageusement pas d'optique de grossissement, celui-ci fonctionnant sur le principe d'imagerie sans lentille.

Le capteur d'images 3 est relié à une unité de traitement UC recevant les images acquises par le capteur d'images. Le capteur d'images 3 est capable de générer plusieurs images à des instants successifs, permettant ainsi de suivre l'évolution des réactions au niveau de chaque puits 10.

Il serait possible d'employer d'autres moyens de détection que le capteur d'images 3, par exemple un microscope classique. La solution de détection par imagerie sans lentille présente l'avantage de pouvoir surveiller un grand nombre de puits simultanément, éventuellement sans mettre en place de moyens mécaniques complexes si tous les éléments du système sont correctement dimensionnés. Elle permet également un traitement d'images simple.

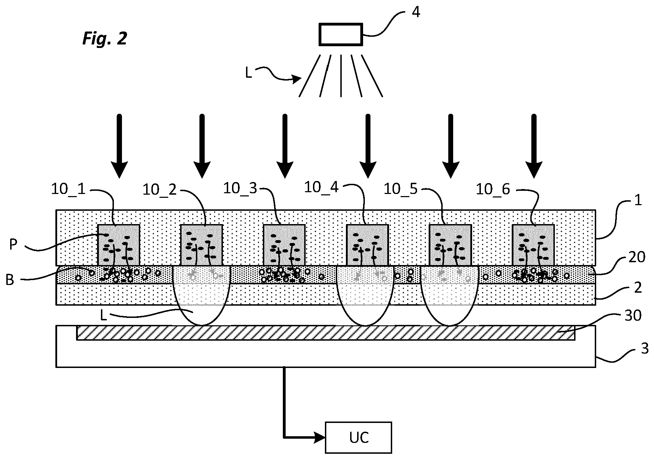

Partant des différents éléments du système tels que décrits ci-dessus, le procédé de détection est mis en œuvre de la manière décrite ci-dessous, en liaison avec la figure 2. La première plaque 1 est par exemple retournée et mise en appui, par sa face ouverte sur les puits contre la face fonctionnelle de la deuxième plaque 2 portant le dépôt 20, de sorte que le contenant de chaque puits 10 vienne au contact du dépôt 20 présent sur la surface fonctionnelle de la deuxième plaque 2. On vient ainsi mettre en contact les phages P présents dans chaque puits 10 avec les bactéries B inoculées dans le dépôt 20 de la deuxième plaque. Les deux plaques 1, 2 sont par exemple maintenues l'une contre l'autre de manière à créer une étanchéité d'un puits à un autre et de permettre d'obtenir une diffusion suffisante des phages P dans le dépôt inoculé de bactéries B.

La perméabilité aux gaz apolaires de la première plaque 1 permet d'assurer la croissance bactérienne, même lorsque les phages P et les bactéries B sont mis en contact, par application de la première plaque 1 portant les phages contre le support des bactéries B.

L'ensemble, par exemple formé des deux plaques, est positionné entre la source de lumière 4 et le capteur d'images 3. Il est par exemple positionné au plus proche de la surface 30 du capteur 3, matérialisée par exemple par une vitre de protection, par exemple sur cette surface ou juste au-dessus. Comme déjà indiqué ci-dessus, il faut noter que le dépôt 20 pourrait être disposé directement sur la surface 30 du capteur 3, celle-ci servant alors de support des bactéries B.

La source de lumière 4 est activée de manière à venir éclairer les différents puits 10. L'éclairage peut permettre d'illuminer tous les puits 10 simultanément ou l'un après l'autre ou par groupe de plusieurs puits. Dans ce dernier cas, des moyens d'actionnement peuvent être prévus pour effectuer un déplacement de l'ensemble formé des deux plaques et/ou de la source de lumière 4.

Le capteur d'images 3 est activé de manière à venir capturer, simultanément ou quasi-simultanément, des images de chaque puits 10.

En l'absence de phages ou d'effets notables des phages P sur la souche bactérienne, les bactéries prolifèrent. Lorsque des phages P présentent une sensibilité particulière pour la souche bactérienne et une activité lytique sur celle-ci, le nombre de bactéries B diminue. L'augmentation ou la diminution du nombre de bactéries B au niveau de chaque puits 10 entraîne alors une variation de la nature et/ou de l'intensité de la lumière transmise à travers chaque puits 10. Les images acquises par le capteur 3 au cours du temps permettent ainsi d'apprécier et de quantifier cette évolution. Autrement dit, au niveau de chaque puits 10, si le phage P présente une activité lytique particulière vis-à-vis de la souche bactérienne, celui-ci aura tendance à venir lyser les bactéries B. L'intensité de la lumière émise par la source de lumière 4 à travers le puits 10 et captée par le capteur d'images augmentera.

En revanche, si le phage P présent dans ledit puits ne présente qu'une faible activité lytique, voire aucune activité lytique pour la souche bactérienne, l'intensité de la lumière émise par la source de lumière 4 à travers le puits 10 et captée par le capteur d'images 3 sera stable ou baissera, du fait de la prolifération des bactéries B. Les principes de détection sont par exemple décrits dans la demande de brevet EP3822360A1**.**

Sur la figure 2, on peut ainsi voir que, au niveau de chaque puits 10, les phages diffusent dans le dépôt 20 inoculé de bactéries B. Certains phages P qui présentent une sensibilité avec la souche bactérienne viennent détruire les bactéries B et d'autres qui n'en ont pas vont cohabiter avec les bactéries B, les bactéries B pouvant alors poursuivre leur prolifération. Sur cette figure 2, les phages P initialement présents dans les puits 10_2, 10_4, 10_5 présentent une sensibilité pour la souche bactérienne et une activité lytique sur les bactéries. Les signaux lumineux transmis à travers ces puits sont d'une intensité plus élevée que ceux transmis à travers les autres puits 10_1, 10_3, 10_6, pour lesquels les phages P (en composition et/ou en concentration) ne présentent pas une sensibilité particulière vis-à-vis de la souche bactérienne. Au niveau des puits 10_1, 10_3, 10_6, les signaux lumineux sont en effet atténués par les composés (bactéries + phages) présents.

Le même principe s'applique avec une deuxième plaque compartimentée.

Pour imager tous les puits, il faut noter qu'il est également possible de déplacer le capteur d'images 3 et/ou l'ensemble première plaque/deuxième plaque, s'il s'avère que la surface de capture 30 n'est pas suffisante pour visualiser tous les puits 10 en une seule image.

L'invention permet de tester rapidement l'activité lytique de phages P vis-à-vis d'une souche bactérienne. Le test peut être réalisé en quelques heures seulement.

A titre d'exemple, en utilisant un capteur d'image de 864mm2, il a été possible de surveiller soixante-six puits de manière simultanée. Bien entendu, il serait possible d'augmenter le nombre de puits sur une même plaque, et/ou d'augmenter la surface du capteur et/ou même d'utiliser plusieurs capteurs juxtaposés.

Il faut également noter que le principe de l'invention permet de suivre en temps réel l'activité lytique des phages sur les bactéries. L'information cinétique issue de l'acquisition en temps réel apporte ainsi une couche d'information supplémentaire.

L'invention présente de nombreux avantages, parmi lesquels :
- Le multiplexage des mesures d'activité de phages sur des souches bactériennes,
- L'utilisation de composants classiques tels que plaque à puits, capteur d'images, source de lumière,
- Un procédé facile à mettre en œuvre et capable de fournir des résultats rapidement,
- Un procédé fiable car ne nécessitant que peu de manipulations,
- Un procédé permettant un suivi en continu, pouvant donner des résultats plus rapides lorsque mis en œuvre dans une étuve.

## Revendications

1. Procédé de détection d'une activité lytique d'un ou plusieurs types de phages (P) ou d'activité d'un ou plusieurs antibiotiques vis-à-vis d'une souche bactérienne, les phages (P) ou antibiotiques étant éventuellement distincts entre eux en composition et/ou concentration, **caractérisé en ce qu'**il consiste à :
- Utiliser une première plaque (1) à plusieurs puits, chaque puits (10) étant réalisé dans un matériau transparent aux signaux lumineux (L) émis par une source de lumière (4) et perméable aux gaz apolaires, chaque puits (10) recevant un échantillon contenant des phages ou des antibiotiques,
- Réaliser au moins un dépôt (20) sur une zone transparente d'un support, le dépôt (20) contenant des bactéries (B) de la souche bactérienne,
- Appliquer la première plaque (1) contre le support de sorte que chaque échantillon présent dans chaque puits (10) vienne au contact dudit dépôt (20),
- Disposer l'ensemble formé de la première plaque (1) et le support entre une source de lumière (4) et des moyens de détection de lumière,
- Emettre à l'aide de la source de lumière (4) des signaux lumineux (L) à travers chaque puits (10),
- Pour chaque puits, déterminer à l'aide des moyens de détection l'activité ou l'absence d'activité du phage ou de l'antibiotique vis-à-vis de ladite souche bactérienne en tenant compte de l'intensité des signaux lumineux transmis et/ou de sa variation au cours du temps.

2. Procédé selon la revendication 1, **caractérisé en ce que** les phages ou antibiotiques sont présents dans chaque puits sous la forme d'un hydrogel.

3. Procédé selon la revendication 1 ou 2, **caractérisé en ce que** les bactéries (B) sont dispersées dans un gel déposé sur ladite deuxième plaque (2) de manière à former ledit dépôt (20).

4. Procédé selon l'une des revendications 1 à 3, **caractérisé en ce que** le support est réalisé sous la forme d'une deuxième plaque (2) compartimentée à l'aide de cloisons étanches de manière à former plusieurs compartiments distincts, ledit dépôt étant subdivisé en plusieurs dépôts distincts ajoutés dans chaque compartiment de la deuxième plaque.

5. Procédé selon la revendication 4, **caractérisé en ce que** la première plaque (1) est maintenue contre ladite deuxième plaque (2) de sorte que les phages (P) ou antibiotiques contenus dans chaque puits viennent au contact avec un dépôt placé dans un compartiment distinct de la deuxième plaque.

6. Procédé selon l'une des revendications 1 à 5, **caractérisé en ce que** la première plaque (1) est retournée et déplacée pour venir s'appliquer par une face ouverte sur lesdits puits (10) contre une face fonctionnelle du support portant le dépôt (20).

7. Procédé selon l'une des revendications 1 à 5, **caractérisé en ce que** le support est retourné de manière à venir s'appliquer par une face fonctionnelle portant ledit dépôt (20) contre une face de la première plaque (1) ouverte sur lesdits puits (10).

8. Procédé selon l'une des revendications 1 à 7, **caractérisé en ce qu'**il comporte une étape de déplacement relatif de l'ensemble formé par la première plaque (1) et le support par rapport aux moyens de détection en vue de transmettre les signaux lumineux (L) à travers chaque puits (10) de la première plaque (1).

9. Procédé selon l'une des revendications 1 à 8, **caractérisé en ce que** les moyens de détection comportent un capteur d'images (3).

10. Procédé selon la revendication 9, **caractérisé en ce que** le capteur d'images (3) est configuré pour acquérir une première image à un premier instant et une deuxième image à un deuxième instant postérieur et **en ce qu'**une unité de traitement est configurée pour déterminer que la souche bactérienne est considérée comme :
- Peu ou pas sensible aux phages ou antibiotiques lorsque l'intensité des signaux lumineux détectés diminue entre lesdits deux instants, ou
- Sensible aux phages ou antibiotiques lorsque l'intensité des signaux lumineux détectés ne diminue pas ou augmente entre lesdits deux instants.

11. Procédé selon la revendication 9 ou 10, **caractérisé en ce que** le capteur d'images (3) coopère avec la source de lumière (4) selon un principe d'imagerie sans lentille.

12. Procédé selon l'une des revendications 1 à 11, **caractérisé en ce que** la source de lumière est configurée pour émettre dans une bande spectrale comprise entre 500nm et 600nm.

## Patentansprüche

1. Verfahren zur Detektion einer lytischen Aktivität eines oder mehrerer Typen von Phagen (P) oder von Aktivität eines oder mehrerer Antibiotika gegenüber einem Bakterienstamm, wobei die Phagen (P) oder Antibiotika gegebenenfalls von der Zusammensetzung und/oder Konzentration her untereinander verschieden sind, **dadurch gekennzeichnet, dass** es in Folgendem besteht:
- Verwenden einer ersten Platte (1) mit mehreren Vertiefungen, wobei jede Vertiefung (10) aus einem Material ausgeführt ist, das für die von einer Lichtquelle (4) emittierten Lichtsignale (L) transparent ist und für apolare Gase durchlässig ist, wobei jede Vertiefung (10) eine Phagen oder Antibiotika enthaltende Probe aufnimmt,
- Ausführen mindestens einer Schicht (20) auf einem transparenten Bereich eines Trägers, wobei die Schicht (20) Bakterien (B) des Bakterienstamms enthält,
- Anlegen der ersten Platte (1) an den Träger, so dass jede in jeder Vertiefung (10) vorhandene Probe in Kontakt mit der Schicht (20) gelangt,
- Anordnen der aus der ersten Platte (1) und dem Träger gebildeten Anordnung zwischen einer Lichtquelle (4) und Lichtdetektionsmitteln,
- Emittieren von Lichtsignalen (L) durch jede Vertiefung (10) hindurch mithilfe der Lichtquelle (4),
- Bestimmen, für jede Vertiefung, der Aktivität oder des Nichtvorliegens von Aktivität des Phagen oder des Antibiotikums gegenüber dem Bakterienstamm mithilfe der Detektionsmittel unter Berücksichtigung der Intensität der transmittierten Lichtsignale und/oder ihrer Änderung im Laufe der Zeit.

2. Verfahren nach Anspruch 1, **dadurch gekennzeichnet, dass** die Phagen oder Antibiotika in jeder Vertiefung in Form eines Hydrogels vorhanden sind.

3. Verfahren nach Anspruch 1 oder 2, **dadurch gekennzeichnet, dass** die Bakterien (B) in einem Gel dispergiert sind, das auf die zweite Platte (2) aufgetragen wird, so dass es die Schicht (20) bildet.

4. Verfahren nach einem der Ansprüche 1 bis 3, **dadurch gekennzeichnet, dass** der Träger in Form einer zweiten Platte (2) ausgeführt ist, die mithilfe von dichten Trennwänden kompartimentiert ist, so dass mehrere verschiedene Kompartimente gebildet werden, wobei die Schicht in mehrere verschiedene Schichten unterteilt wird, die in jedem Kompartiment der zweiten Platte hinzugefügt werden.

5. Verfahren nach Anspruch 4, **dadurch gekennzeichnet, dass** die erste Platte (1) gegen die zweite Platte (2) gehalten wird, so dass die in jeder Vertiefung enthaltenen Phagen (P) oder Antibiotika in Kontakt mit einer in einem anderen Kompartiment der zweiten Platte angeordneten Schicht gelangen.

6. Verfahren nach einem der Ansprüche 1 bis 5, **dadurch gekennzeichnet, dass** die erste Platte (1) gewendet und verlagert wird, um mit einer an den Vertiefungen (10) offenen Seite an eine die Schicht (20) tragende funktionelle Seite des Trägers angelegt zu werden.

7. Verfahren nach einem der Ansprüche 1 bis 5, **dadurch gekennzeichnet, dass** der Träger gewendet wird, so dass er mit einer die Schicht (20) tragenden funktionellen Seite an eine an den Vertiefungen (10) offene Seite der ersten Platte (1) angelegt wird.

8. Verfahren nach einem der Ansprüche 1 bis 7, **dadurch gekennzeichnet, dass** es einen Schritt des relativen Verlagerns der aus der ersten Platte (1) und dem Träger gebildeten Anordnung in Bezug auf die Detektionsmittel umfasst, um die Lichtsignale (L) durch jede Vertiefung (10) der ersten Platte (1) hindurch zu transmittieren.

9. Verfahren nach einem der Ansprüche 1 bis 8, **dadurch gekennzeichnet, dass** die Detektionsmittel einen Bildsensor (3) umfassen.

10. Verfahren nach Anspruch 9, **dadurch gekennzeichnet, dass** der Bildsensor (3) dazu ausgestaltet ist, ein erstes Bild zu einem ersten Zeitpunkt und ein zweites Bild zu einem späteren zweiten Zeitpunkt zu erfassen, und dass eine Verarbeitungseinheit dazu ausgestaltet ist zu bestimmen, dass der Bakterienstamm betrachtet wird als:
- kaum oder nicht empfindlich gegenüber Phagen oder Antibiotika, wenn die Intensität der detektierten Lichtsignale zwischen den beiden Zeitpunkten abnimmt, oder
- empfindlich gegenüber Phagen oder Antibiotika, wenn die Intensität der detektierten Lichtsignale zwischen den beiden Zeitpunkten nicht abnimmt oder zunimmt.

11. Verfahren nach Anspruch 9 oder 10, **dadurch gekennzeichnet, dass** der Bildsensor (3) mit der Lichtquelle (4) nach einem Prinzip einer linsenlosen Bildgebung zusammenwirkt.

12. Verfahren nach einem der Ansprüche 1 bis 11, **dadurch gekennzeichnet, dass** die Lichtquelle dazu ausgestaltet ist, in einem Spektralband zwischen 500 nm und 600 nm zu emittieren.

## Claims

1. Method for detecting a lytic activity of one or more types of phages (P) or of the activity of one or more antibiotics with respect to a bacterial strain, the phages (P) or antibiotics optionally being distinct from each other in terms of composition and/or concentration, **characterized in that** it consists in:
- using a first multiwell plate (1), each well (10) being made of a material transparent to the light signals (L) emitted by a light source (4) and permeable to apolar gases, each well (10) receiving a sample containing phages or antibiotics,
- making at least one deposit (20) on a transparent area of a support, the deposit (20) containing bacteria (B) of the bacterial strain,
- applying the first plate (1) against the support so that each sample in each well (10) comes into contact with said deposit (20),
- placing the assembly formed by the first plate (1) and the support between a light source (4) and light detection means,
- emitting light signals (L) through each well (10) using the light source (4),
- for each well, determining, using the detection means, the activity or absence of activity of the phage or antibiotic with respect to said bacterial strain, taking into account the intensity of the transmitted light signals and/or the variation thereof over time.

2. Method according to Claim 1, **characterized in that** the phages or antibiotics are present in each well in the form of a hydrogel.

3. Method according to Claim 1 or 2, **characterized in that** the bacteria (B) are dispersed in a gel deposited on said second plate (2) so as to form said deposit (20).

4. Method according to one of Claims 1 to 3, **characterized in that** the support is made in the form of a second plate (2) compartmentalized by means of leaktight partitions so as to form several distinct compartments, said deposit being subdivided into several distinct deposits added to each compartment of the second plate.

5. Method according to Claim 4, **characterized in that** the first plate (1) is held against said second plate (2) so that the phages (P) or antibiotics contained in each well come into contact with a deposit placed in a compartment separate from the second plate.

6. Method according to one of Claims 1 to 5, **characterized in that** the first plate (1) is turned upside down and moved so as to come to bear, via an open face on said wells (10), against a functional face of the support carrying the deposit (20).

7. Method according to one of Claims 1 to 5, **characterized in that** the support is turned upside down so as to come to bear, via a functional face carrying said deposit (20), against a face of the first plate (1) open on said wells (10).

8. Method according to one of Claims 1 to 7, **characterized in that** it comprises a step of relative displacement of the assembly formed by the first plate (1) and the support relative to the detection means for the purpose of transmitting the light signals (L) through each well (10) of the first plate (1).

9. Method according to one of Claims 1 to 8, **characterized in that** the detection means comprise an image sensor (3).

10. Method according to Claim 9, **characterized in that** the image sensor (3) is configured to acquire a first image at a first time and a second image at a later second time and **in that** a processing unit is configured to determine that the bacterial strain is considered to be:
- barely or not at all sensitive to the phages or antibiotics when the intensity of the detected light signals decreases between said two times, or
- sensitive to the phages or antibiotics when the intensity of the detected light signals does not decrease or increases between said two times.

11. Method according to Claim 9 or 10, **characterized in that** the image sensor (3) cooperates with the light source (4) according to a lensless imaging principle.

12. Method according to one of Claims 1 to 11, **characterized in that** the light source is configured to emit in a spectral band between 500 nm and 600 nm.
